# EUROPEAN PATENT APPLICATION

(11) **EP 2 130 904 A2**
(43) Date of publication of application: **09.12.2009**
(21) Application number: 09161911.4
(22) Date of filing: 04.06.2009
(51) Int. Cl.: C12M 3/00, C12N 5/00

(54) **Bioreactor, particularly for generating engineered tissues**

(30) Priority: 05.06.2008 IT TO20080426
(71) Applicant: Università degli Studi di Genova, 16126 Genova (IT)
(72) Inventor: Scaglione, Silvia, I-16156, GENOVA (IT); Quarto, Rodolfo, I-16035, RAPALLO (Genova) (IT); Benatti, Umberto, I-16125, GENOVA (IT); Badano, Roberto, I-16137, GENOVA (IT)
(74) Representative: Deambrogi, Edgardo

(57) **Abstract**

A culture chamber (10) for a bioreactor is disclosed, comprising a container (11), a first grasping member (21), and a second grasping member (22) which are adapted to hold respective opposite ends of a support (S) for the culture of cells; and a handling shaft (41) which is integral with the first grasping member. The container comprises a protective bell (12) and a cover (13), in which the protective bell is applied from the bottom and removably to the cover, and the cover has a tubular projection (17), inside which at least part of the first grasping member handling shaft is housed and enclosed. A driving member (44) is further provided, which is arranged around the tubular projection and adapted to be connected to outer actuators, which is made magnetically integral to the handling shaft. The second grasping member is connected to the cover via a support (31) descending from the latter.

## Description

The present invention generally relates to bioreactors, particularly for the generation of engineered tissues.

Such instruments allow subjecting (stem, progenitor, differentiated) cells, which are seeded on suitable three-dimensional biomaterials, to controlled mechanical stimuli (torsion, traction, compression).

More particularly, the present invention relates to a culture chamber for a bioreactor, comprising
a container;
holding means arranged within said container, and adapted to hold a support for the culture of cells, said holding means comprising a first grasping member and a second grasping member which are aligned according to an assembly direction of the support, and adapted to hold respective opposite ends of the culture support; and
connecting means, adapted to connect said first grasping member to external actuating means to impart a desired mechanical stress to the culture cells, said connecting means comprising a handling shaft which is integral with the first grasping member, and extending along the assembly direction of the support.

Tissue engineering has been defined as the science of persuading a body to reconstruct those tissues and/or organs that are not capable of spontaneously regeneration. In one of the possible approaches to this aim, living cells are used to generate *ex-vivo* implantable structures, which could cover losses of tissues (e.g., as a result of trauma or tumour surgery), or replace non-functional organs (e.g., to overcome the problem of the reduced availability of organs from healthy donors). Anyway, the possible impact of this field is much wider: in the future, the engineered tissues could not only reduce the need for organs to be transplanted, but may also constitute model systems of tissues *ex vivo,* which would allow speeding up the development of new medicaments and the research for new therapies, in order to improving the tissue regeneration.

In order to engineering tissues *in vitro,* specific types of cells (stem, progenitor, differentiated cells) are cultured on bio-active degradable supports having the chemical-physical characteristics that allow the cells themselves to differentiate and generate three-dimensional tissue structures (the so-called "constructs"). Inducing cells to form tissues in a reproducible manner is the fundamental problem of tissue engineering.

In addition to stimuli of a biochemical nature, it has been noticed that the cell growth, and especially the spatial organization of the cells (that is essential for the structural tissues) are also affected by stimuli of a mechanical origin.

Thereby, the bioreactor systems allow promoting the cell growth in the desired physiological specialization. At the same time, the bioreactor systems allow automatizing the tissue regeneration process, and switching to the use of the regenerative medicine in the routine clinical practice, thus ensuring that the techniques used in the laboratory are standardizable and easily reproducible.

Although, in recent years, a number of bioreactor systems have been patented and/or published on scientific journals of the field, few are the available applications relating to the large-scale generation of engineered tissues.

Therefore, object of the present invention is to provide a bioreactor characterized by a high use versatility and ease of use, and which further drastically reduces the contamination problems within the culture chamber.

That object is achieved according to the invention by a culture chamber for a bioreactor, in which
said container comprises a protective bell and a cover, in which the protective bell is applied from the bottom and removably to the cover, and said cover has a tubular projection inside which at least part of the handling shaft of the first grasping member is housed and enclosed;
said connecting means further comprise a driving member which is arranged around said tubular projection and adapted to be connected to said external actuating means, said driving member being made magnetically integral to said handling shaft, and
said second grasping member is connected to the cover by a support descending from the latter.

In the above-mentioned chamber, the bell closure system allows simplifying the assembly step of the substrate, by operating with a completely wide-open chamber. At the same time, the bell system drastically reduces the sterility problems due to the circulating fluid flows. In fact, the fluid is confined in a closed system (the bell), without any possibility of liquid losses.

Furthermore, the magnetic connection between the handling shaft of the first adjustable holding member and the outer driving member ensures a high accuracy, ease of use, and especially drastically reduces the contamination problems within the chamber, since the motion transmission does not provide communications with the exterior.

Furthermore, the proposed system can be tailored to any type of substrate on which the cells are being cultured: in fact, it is possible to provide for setting the operative length (substrate length) by modifying the initial height position of the grasping members. By adopting a self-centering system for the grasping members, it is possible to tailor the bioreactor to different diameters of the substrates, while maintaining the alignment with the handling shaft (a necessary condition in order to subject the substrate to torsion).

Depending on the required experimental conditions, it is possible to further add a perfusion system to the mechanical stimulation, for the recirculation of the fluid flows of the culture system (which is, e.g., driven by a peristaltic pump).

It is a further object of the invention a bioreactor comprising a culture chamber according to the invention.

Preferred embodiments of the invention are defined in the dependant claims.

Further features and advantages will result from the following description, with reference particularly to the annexed drawings, given by way of non-limiting example only, in which
Fig. 1 is a general diagram of a bioreactor according to the invention;
Fig. 2 is a cross-sectional view of a culture chamber of the bioreactor of Fig. 1;
Figs. 3a-3c are cross-sectional, front, plane views, respectively, of a grasping member of the bioreactor of Fig. 1;
Figs. 3d-3e are perspective views of components of the grasping member of the Figs. 3a-3c;
Fig. 4 is a block diagram of the electronic control system of the bioreactor of Fig. 1; and
Fig. 5 is a block diagram of the on-line control of the tissue growth in the bioreactor of Fig. 1.

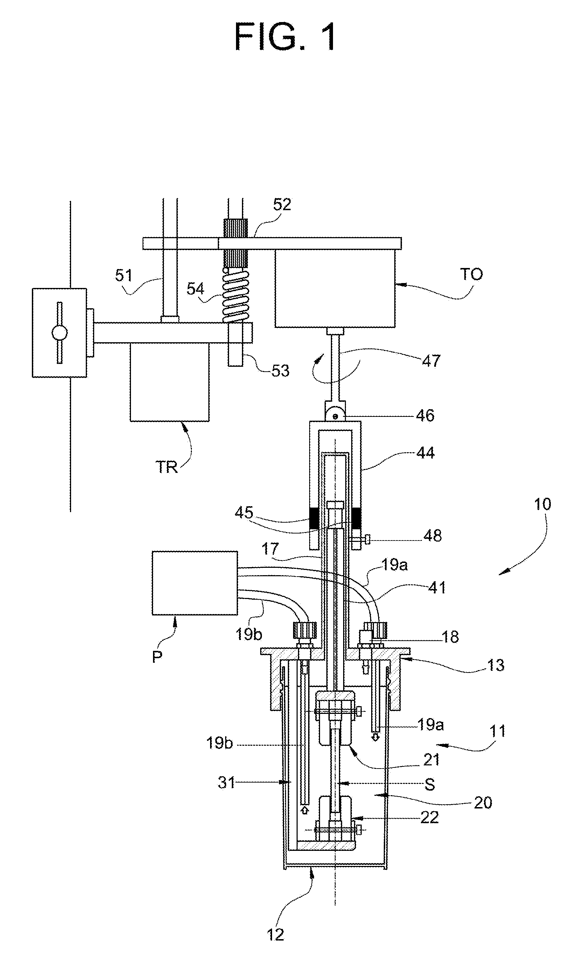

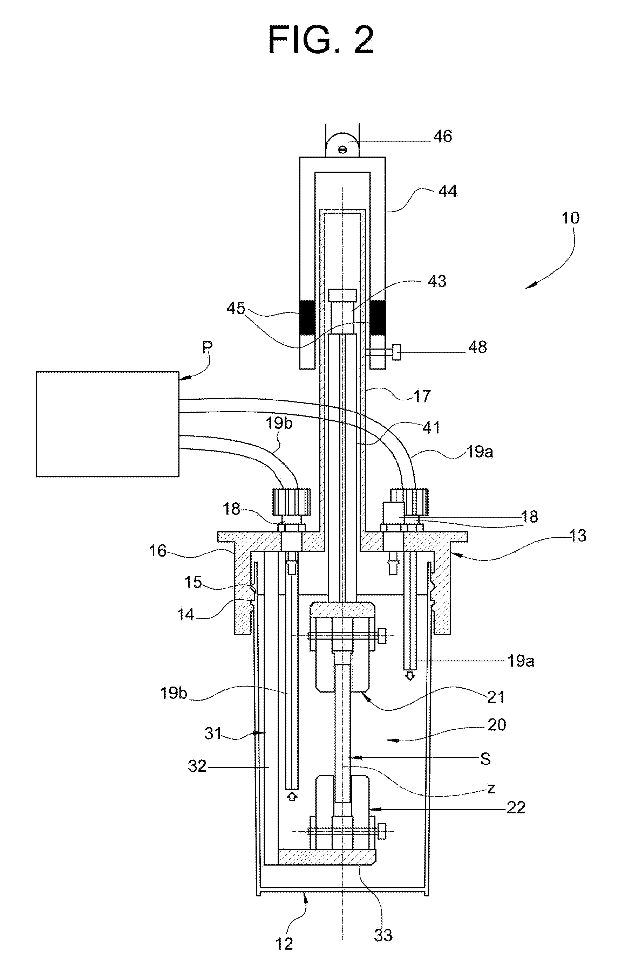

With reference to Figs. 1 and 2, a culture chamber is generally indicated with 10.

The chamber 10 comprises a container 11, composed of a protective bell 12 and a cover 13. The protective bell 12 is applied from below and removably to the cover 13. Particularly, the bell 12 has a thread 14 on the open end thereof, which is adapted to engage a complementary thread 15 that is obtained on a collar portion 16 of the cover 13. Preferably, the chamber 12 is in polymeric material, particularly a transparent one, and it is preferably disposable and prepared in a sterile condition.

On the cover 13, which is preferably made of Teflon®, a tubular projection 17 is obtained, the inner cavity of which is in communication with the interior of the culture chamber 10, while it is insulated relative to the external environment, as the tubular projection 17 wall is free from openings. The function of such projection 17 will be elucidated herein below.

On the cover 13, connectors 18 are further arranged, for example, of the Luer-lock type, for the installation of sensors (not shown) adapted to monitor the operative conditions of the bio-reactor, such as pH, oxygen, temperature sensors, etc., and/or for the connection of pipes 19a, 19b, the function of which will be described herein below.

Within the chamber 10, holding means 20 are arranged, which are adapted to hold a support S for the culture of cells. The holding means 20 comprise a first grasping member 21 and a second grasping member 22 which are aligned according to an assembly direction z of the support, and adapted to hold respective opposite ends of the culture support S.

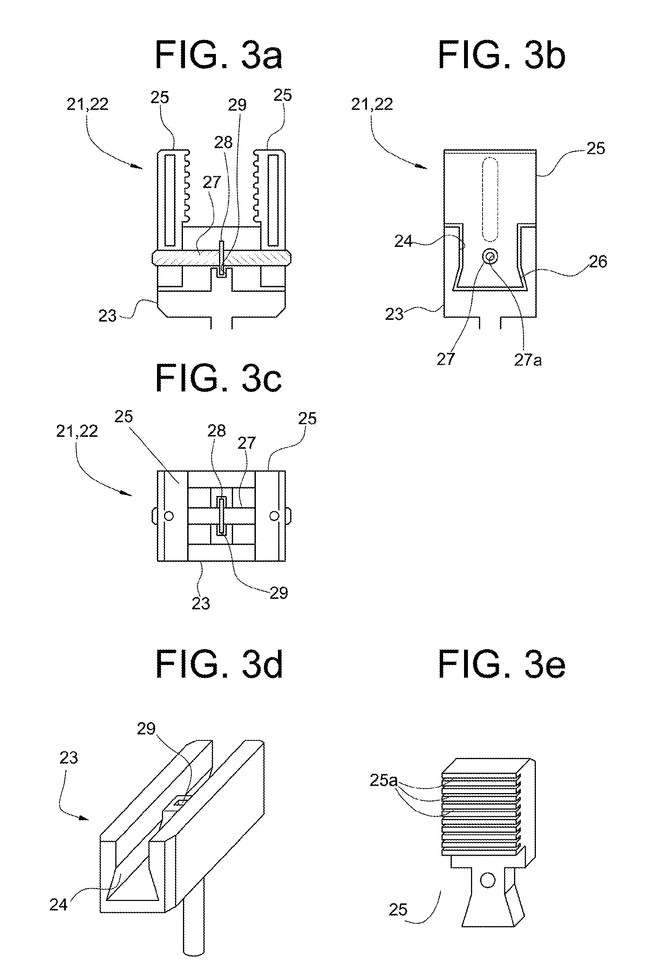

The grasping members 21 and 22 are illustrated in more detail in Figs. 3a-3e. Particularly, each grasping member is formed by a clamp, comprising a base block 23, on a side of which a guide groove 24 is obtained, on which two jaws 25 are slidably mounted, that are provided with formations 26 (in the shown example, in the form of a dovetail) for a shape-fit coupling with the guide groove 24. The positioning and clamping of the jaws are adjusted by an adjustment screw 27, which has the two halves thereof with inverted thread one relative to the other, in order to obtain a self-centering system. The adjustment screw is axially constrained in the central part of the clamp 21, 22, via a central stop element 28 that is integral thereto, and introduced in a corresponding seat 29 obtained in the base block 23. In order to allow for the clamp 21, 22 adjustment, the adjustment screw 27 is provided at an end of an actuating head 27a, for example, with Allen notch. The jaws 25 have a relatively wide grasping surface, provided with indentations 25 to distribute the force applied and to optimize the grasp on the support S. The adjustment screw 27 is made of a material having a thermal expansion coefficient greater than the remaining material constituting the clamp, so as to prevent the screw from loosening with a prolonged operation. In Figs. 3d and 3e, the base block 23 one of the jaws 25 of the clamps are singularly illustrated.

The second grasping member (clamp) 22 is connected to the cover 13 via a support 31 descending from the latter. Such support 31 comprises a vertical rod 32, secured at an end to the cover 13 underside, and a cantilever member 33, which is secured at the other end of the vertical rod 32, which supports the second grasping member 22.

To impart a desired mechanical stress to the cells anchored to the support S, actuating means TO, TR are provided, that are arranged at the outside of the culture chamber 10, which will be described in more detail herein below. Therefore, the first grasping member 21 is operatively connected to such actuating means TO, TR through a handling shaft 41, that is coaxial with the assembly direction z of the support. At an end of such handling shaft 41, the first grasping member 21 is adjustably mounted in the axial position. In order to be able to perform such adjustment, a loosening of a locking screw 48 shall be performed, which screw will be described in more detail herein below.

At the furthermost end from the first grasping member 21, a ferromagnetic element 43 is splined on the handling shaft 41, the function of which will be elucidated herein below. The handling shaft 41 is at least partially housed and enclosed within the tubular projection 17 of the cover 13. In order to transmit the motion generated by the actuating means TO, TR to the handling shaft 41, a driving member 44 is provided, which is arranged around the tubular projection 17 and coaxial with the shaft 41, and magnetically coupled to the latter. To this end, permanent magnets 45 are arranged on the driving member, which are magnetically coupled to the ferromagnetic element 43 of the handling shaft 41. Preferably, such magnets are in neodymium alloy (NdFeB), which confers particularly characteristics, i.e. a high attraction with the smallest dimensions. Therefore, the shaft 41 is free to rotate and swing, giving the support S and the cells on the support (the construct) the appropriate torsion and/or tension/compression stimuli.

The use of a magnetic system for the coupling of the handling shaft to the exterior ensures a high accuracy, ease of use, and drastically reduces the contamination problems of the culture chamber, since the motion transmission does not provide communications with the exterior. Furthermore, this allows avoiding the use of bellows, gaskets, O-rings, which may cause frictions and/or internal forces, preventing the achievement of a good signal to noise ratio in the control step of the motor.

In order to allow releasing the chamber 10 from the actuating means TO, TR, a releasable clutch 46 is provided on the driving member 44, through which the driving member 44 is releasably connected to an output shaft 47 of such actuating means TO, TR. In order to get security of the system after the clutch 46 release, a locking screw 48 is provided on the driving member 44, which is adapted to lock the driving member 44 to the tubular projection 17 of the cover 13, so as to stop the first grasping member 21 in the rest position thereof. Therefore, the chamber 10, once it has been withdrawn, can be positioned on a suitable stand (not shown), allowing easily operating in a sterile environment (under a hood).

In those conditions, it is also possible to operate with the axial adjustment of the first grasping member 21. By slightly loosening the locking screw 48, in fact, it is possible to act on the driving member 44 in order to make it to slide along the tubular projection 17, consequently shifting the handling shaft 41 along the axial direction thereof, and then adjusting the position of the first grasping member 21.

The actuating means TO, TR comprise two stepper motors, which may operate separately or paired to provide the required stimuli (torsion and traction) to the support S. The first motor, indicated with TR, is provided with a rotor ending with a threaded pin 51, which provides for the displacement in the vertical direction, through a screwed transmission, the second motor TO which is secured on a platform 52 sliding on guides 53. A suspension (spring) 54 arranged at the guides 53 allows balancing the system from the point of view of the load. The second motor TO imparts the rotation to the driving member 44, and then the torsion to the support S, through the output shaft 47.

As known, the stepper motors manage in an optimal way all the applications requiring accuracy in the angular displacement and rotation speed. Furthermore, with such motors it is possible to maintain the shaft stationary in a balance position: in fact, if they are powered, they simply lock in a precise angular position.

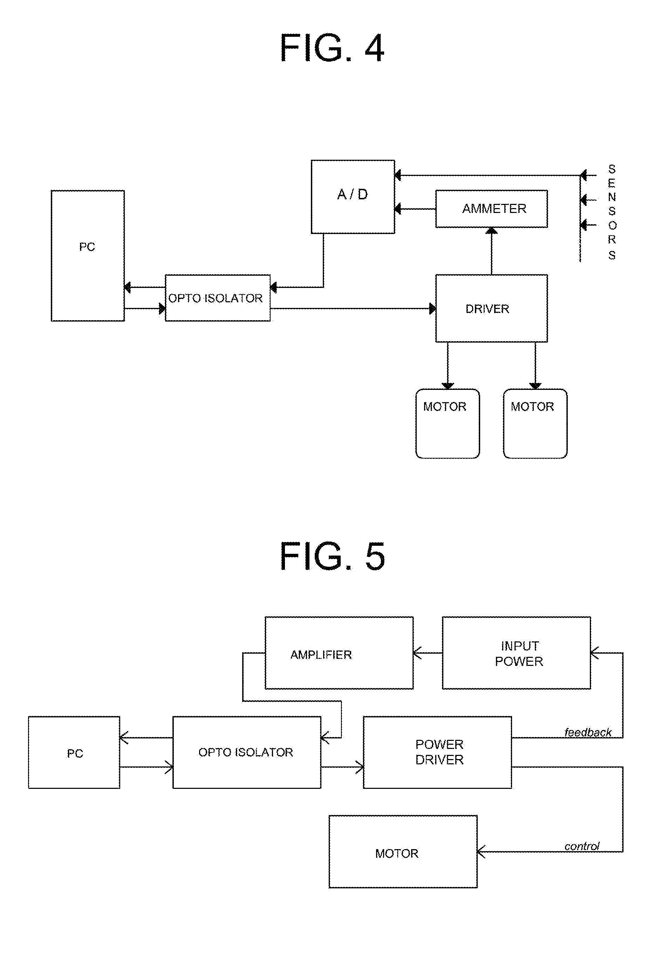

The block diagram of an electronic control system of the bioreactor is shown in Fig 4, while the block diagram of the on-line control of the tissue growth in the bioreactor is shown in Fig 5.

The above-mentioned electronic system drives the two stepper motors TO, TR in order to obtain the rotary motion and the linear motion, respectively, and to subject the cells seeded on the support to torsion and traction stimuli.

In order to obtain a rotation, it is necessary to send a series of current pulses to the motor, according to a suitable sequence, so as to move, through successive steps, the desired position.

By simply counting the pulses and by setting the frequency thereof, it is possible to make the shaft rotate to the desired position and at the desired speed, since the balance positions of the shaft are mechanically determined with extreme accuracy.

Thus, the stepper motor allows the implementation of computer-controlled precision operations in open loop, i.e. without using position or speed sensors. Thus, the circuits can be relatively simple from both the implementation and designing point of view, and no particular calculation power is required to the controller (PC).

The above-mentioned electronic system can acquire on-line the elasticity (stiffness) increase values of the construct during the cell culture (feedback mode). In order to detect the dynamometric data, the same actuators TO, TR are used as transducers. In detail, the measurement of the elasticity (stiffness) variations of the construct takes place on the basis on the reading of the electrical current absorption variation (Δi), and thus of power consumption (ΔP), of the two motors.

By employing the sensors during the cell culture, the electronic system is capable of detecting the temperature, pH, oxygen variations, etc., and of modifying on-time the sequence of mechanical stimulation set for the construct, according to the recorded readings.

According to the required experimental conditions, it is possible to add a perfusion system to the mechanical stimulation, for the recirculation of the fluid flows of the culture system. Such system can be implemented by arranging the tubes 19a and 19b as the inlet tube and the outlet tube, respectively, in order to introduce and withdraw the recirculation fluid in/from the chamber 10, and connecting such tubes to a pump P, for example, a peristaltic pump.

The transportation of gases, nutrients, metabolites, and growth factors is at the core of the cellular physiology, and is capable of determining to a great extent the structure and function of the tissues. The most direct method to increase the *in-vitro* transfer of mass is to induce a flow in the culture medium. The movement of the culture medium generates a dynamic shear at the construct surface, that increases the mass transfer. It has been proven that an efficient mixing is capable of improving the composition and mechanical properties of the engineered tissues, and increasing the cell density and the matrix synthesis from cells seeded on the support S.

With the present invention it is possible to implement a bioreactor which is considerably compact and characterised by a high use practicality and portability, and therefore which is adapted to a routine clinical use. For example, the inventors have produced a prototype with reduced dimensions, with a useful volume below 150 cm³.

For the control of process temperatures, the bioreactor can be associated to a thermostatic system composed of a Dewar vessel chamber, in which the culture chamber 10 is introduced. Internally to such Dewar chamber, an infrared heating system is provided. Thermal radiations radiatively merge to the internal chamber, where the culture chamber 10 is present. Here, they are entrapped by the reflecting wall of the Dewar chamber. Therefore, the heat contribution to keep a temperature of 37 degrees is of a very limited extent. This system allows ensuring the required experimental conditions, while avoiding the use of a much more expensive and complex dedicated incubator for the culture of tissues.

It shall be apparent that, the principle of the finding being understood, the construction details and the embodiments will be able to be widely varied compared to what has been described and illustrated by way of example only, without thereby departing from the scope of the present invention.

## Claims

1. A culture chamber (10) for a bioreactor, comprising
a container (11);
holding means (20) arranged within said container, and adapted to hold a support (S) for the culture of cells, said holding means comprising a first grasping member (21) and a second grasping member (22) which are aligned according to an assembly direction (z) of the support, and adapted to hold respective opposite ends of the culture support; and
connecting means, adapted to connect said first grasping member to outer actuating means (TO, TR) in order to impart a desired mechanical stress to the culture cells, said connecting means comprising a handling shaft (41) which is integral with the first grasping member, and extending along the assembly direction of the support;
**characterized in that**
said container comprises a protective bell (12) and a cover (13), in which the protective bell is applied from the bottom and removably to the cover, and said cover has a tubular projection (17) inside which at least part of the handling shaft of the first grasping member is housed and enclosed;
said connecting means further comprise a driving member (44) arranged around the tubular projection and adapted to be connected to the outer actuating means, said driving member being made magnetically integral to said handling shaft; and
said second grasping member is connected to the cover through a support (31) descending from the latter.

2. The chamber according to claim 1, wherein said grasping members are self-centering, each of them being composed of a clamp, comprising a base block (23), on a side of which a guide groove (24) is obtained, on which two jaws (25) are slidably mounted, which are provided with respective formations (26) for a shape-fit coupling with the guide groove (24), and in which, for the adjustment of the position and clamping of the jaws, an adjustment screw (27) is provided, which has two halves having inverted thread one relative to the other, said adjustment screw being axially constrained in the central part of the clamp by means of a central stop element (28) integrally thereto, which is inserted in a corresponding seat (29) obtained in the base block (23).

3. The chamber according to one of the preceding claims, wherein, at the end of the handling shaft (41) far from the first grasping member, a ferromagnetic member (43) is splined, said ferromagnetic member being magnetically coupled to permanent magnets (45) arranged on the driving member (44).

4. The chamber according to one of the preceding claims, wherein said driving member is adapted to be connected to the outer actuating means (TO, TR) through a releasable clutch (46), a safety locking screw (48) being provided on the driving member (44), which safety locking screw is adapted to be actuated to lock said driving member to the tubular projection (17) of the cover (13) when the driving member is disconnected from the outer actuating means (TO, TR).

5. A bioreactor comprising a chamber according to one of the preceding claims, and further comprising outer actuating means (TO, TR) to impart a desired mechanical stress to the culture cells, wherein said actuating means comprise a first stepper motor (TR), adapted to impart a linear movement to the handling shaft (41), and a second stepper motor (TO), adapted to impart a rotatory movement to the handling shaft (41).

6. The bioreactor according to claim 5, wherein said first motor is provided with a rotor ending with a threaded pin (51), and said second motor is secured on a platform (52) sliding vertically on guides (53), and controlled upon translation by said threaded pin of the first motor through a screwed transmission, and wherein said second motor comprises an output shaft (47) adapted to be coaxially connected to the bioreactor chamber driving member (44).

7. The bioreactor according to one of the claims 5 or 6, further comprising an electronic control system, which is adapted to control said first and second motors according to programmable sequences of current pulses so as to establish angular positions and/or desired speeds of said motors, and adapted to measure elasticity variations of the cell culture on the support (S) on the basis of the reading of the variation of electrical current input of said motors.
